(19) **Europäisches Patentamt / European Patent Office / Office européen des brevets**

(11) **EP 1 804 655 B1**

(12) **FASCICULE DE BREVET EUROPEEN**

(45) Date de publication et mention
de la délivrance du brevet:
**08.07.2015 Bulletin 2015/28**

(21) Numéro de dépôt: **05796030.4**

(22) Date de dépôt: **09.08.2005**

(51) Int Cl.:
**A61B 5/0452** (2006.01)     **A61B 5/0456** (2006.01)

(86) Numéro de dépôt international:
**PCT/FR2005/002056**

(87) Numéro de publication internationale:
**WO 2006/032739 (30.03.2006 Gazette 2006/13)**

(54) **SYSTEME ET PROGRAMME DE TRAITEMENT D'UNE SERIE DES SIGNAUX CARDIAQUES (RR) POUR L'EVALUATION DE LA DOULEUR OU DU STRESS CHEZ UN ETRE VIVANT**

SYSTEM UND PROGRAMM ZUR VERARBEITUNG EINER REIHE VON HERZRHYTHMUSSIGNALEN (RR) ZUR BEURTEILUNG VON SCHMERZ ODER STRESS EINES PATIENTEN

SYSTEM AND PROGRAM FOR PROCESSING A SERIES OF CARDIAC RHYTHM SIGNALS (RR) FOR ASSESSING A PATIENT'S PAIN OR STRESS LEVEL

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **20.09.2004 EP 04370029**

(43) Date de publication de la demande:
**11.07.2007 Bulletin 2007/28**

(73) Titulaires:
• **Centre Hospitalier Regional Universitaire de Lille**
  **59037 Lille Cédex (FR)**
• **UNIVERSITE DE DROIT ET DE LA SANTE DE LILLE 2**
  **59800 Lille (FR)**

(72) Inventeurs:
• **LOGIER, Régis**
  **F-59700 Marcq en Baroeul (FR)**
• **JEANNE, Mathieu**
  **F-59000 Lille (FR)**
• **TAVERNIER, Benoît**
  **F-59130 Lambersart (FR)**

(74) Mandataire: **Matkowska, Franck**
**Matkowska & Associés**
**9, rue Jacques Prévert**
**59650 Villeneuve d'Ascq (FR)**

(56) Documents cités:
**WO-A-03/057034        WO-A1-03/084396**
**WO-A1-2004/016172    US-A- 5 341 811**

## Description

Domaine de l'invention

La présente invention concerne d'une manière générale l'analyse de la variabilité du rythme cardiaque chez un être vivant, et ses applications à l'évaluation de douleur chez un être vivant, notamment un être vivant conscient sous anesthésie locale du type péridurale, ou un être vivant inconscient sous anesthésie générale, et à l'évaluation du stress ressenti par un être vivant.

Art antérieur

D'un point de vue physiologique, le coeur d'un être vivant, isolé de toute influence extérieure, se contracte automatiquement de façon très régulière comme un métronome, sous l'action du noeud sinusal qui génère un influx nerveux indépendant, et par là-même provoque une contraction spontanée du muscle cardiaque. Le coeur n'est toutefois pas isolé, mais est relié au Système Nerveux Autonome (SNA), par l'intermédiaire des systèmes parasympathique et sympathique. Ce système nerveux autonome influe sur l'activité du coeur : le système sympathique accélère le rythme cardiaque, tandis que le système parasympathique le ralentit.

Ainsi, malgré une certaine autonomie, le coeur subit des influences du système nerveux autonome, ce qui permet notamment à l'organisme d'un être vivant d'adapter le rythme cardiaque en fonction de ses besoins, dans des limites toutefois raisonnables. On comprend en conséquence que l'analyse de l'évolution dans le temps du rythme cardiaque, et en particulier des variations du rythme cardiaque (variation de l'intervalle de temps entre deux battements cardiaques) permet d'obtenir une information importante sur l'activité du système cardiaque, et plus particulièrement sur l'activité du système nerveux autonome. Or la connaissance de l'activité du SNA peut être d'une aide précieuse dans l'élaboration d'un diagnostic de bon nombre de situations cliniques. Sur ce sujet, on pourra se référer par exemple à la publication ci-après : Lacroix D, Logier R., Kacet S.,Hazard J-R, Dagano J. (1992) : « Effects of consécutive administration of central and peripheral anticholinergic agents on respiratory sinus arrhythmia in normal subjects, J. of the Autonomic Nervous System », Vol 39, pages 211-218.

Pour étudier ces fluctuations du rythme cardiaque, et par là-même l'activité du SNA, on a déjà depuis 1970 développé différentes techniques d'analyse spectrale d'un signal qui représente l'évolution dans le temps du rythme (ou fréquence) cardiaque instantané, et qui est obtenu après échantillonnage d'un signal bio-électrique analogique, caractéristique du rythme cardiaque d'un être vivant, et dit par la suite signal cardiaque.

Les principaux procédés d'analyse de la variabilité du rythme cardiaque connus à ce jour consistent :

- à acquérir un signal cardiaque, par tout moyen invasif ou non invasif [par exemple, et de manière non exhaustive, acquisition d'un signal électrocardiographique (ECG) au moyen d'un électrocardiographe, ou utilisation d'un capteur de pression sanglante relié à un cathéter introduit dans une artère, ou utilisation d'un capteur de pouls infrarouge],
- à construire à partir de ce signal une série dite RR qui est constituée d'une pluralité d'échantillons ($RR_i$) représentant les intervalles de temps qui séparent deux battements cardiaques successifs,
- et à procéder à une analyse spectrale de la série RR.

Plus particulièrement, l'analyse spectrale d'une série RR issue d'un signal cardiaque est de manière usuelle réalisée en deux étapes principales.

Dans une première étape, on calcule la courbe de densité spectrale de la série RR, par exemple entre 0 et 2Hz, en utilisant différentes méthodes connues. La méthode la plus couramment utilisée consiste à calculer la transformée de Fourier discrète rapide de la série RR, dans des fenêtres temporelles prédéfinies, pondérées au moyen d'une fenêtre de pondération prédéfinie. Il peut s'agir selon la réalisation envisagée d'une fenêtre de pondération rectangulaire, ou encore par exemple d'une fenêtre de pondération de Kaiser, Hamming, Hanning ou Bartlett. Egalement, les fenêtres temporelles de calcul peuvent être prédéfinies et fixes, ou il peut s'agir d'une fenêtre temporelle de calcul, de taille prédéterminée, que l'on fait glisser dans le temps. Par exemple, la transformée de Fourrier est effectuée dans une fenêtre temporelle glissante de 256 secondes, appliquée sur la série RR, et soumise à une pondération de Kaiser pour limiter les effets de bord dus au fenêtrage.

Dans une seconde étape, à partir de la courbe de densité spectrale obtenue à l'issue de la première étape, on calcule automatiquement les puissance spectrales (aires sous la courbe de densité spectrale) entre des bornes de fréquences prédéterminées, et éventuellement réglables par un utilisateur. Ces calculs de puissance spectrale permettent d'obtenir des informations quantitatives, qui sont caractéristiques de l'activité du Système Nerveux Autonome (SNA), et constituent ainsi un moyen d'investigation et d'analyse de la régulation cardiaque par le SNA.

La méthode d'analyse spectrale précitée présente toutefois plusieurs inconvénients.

Une série RR est non stationnaire. Par conséquent, l'application d'une transformée de Fourier sur ce type de série non stationnaire donne des résultats qui sont imprécis, voire le cas échéant erronés, et que l'on ne peut pas interpréter avec certitude. Le calcul de la courbe de densité spectrale par transformée de Fourrier rapide (ou équivalent), est relativement lourd en termes de puissance de calcul et/ou temps de calcul, ce qui rend à ce jour cette méthode d'analyse spectrale inadaptée et difficile à mettre en oeuvre en temps réel notamment dans des systèmes embarqués.

Egalement, pour obtenir une résolution fréquentielle acceptable, la transformée de Fourrier rapide doit être calculée dans des fenêtres temporelles relativement larges (par exemple 256s), ce qui correspond à un nombre important d'échantillons, de la série RR. Il en découle que cette méthode d'analyse spectrale s'accompagne d'un effet mémoire qui retarde la prise en compte d'un changement intervenu dans la série RR.

WO-A-03 057 034 décrit un procédé d'évaluation du risque d'arythmie cardiaque chez un patient consistant à effectuer la mesure de la santé cardiaque ou cardio-vasculaire de ce patient.

WO-A-03 084 396 décrit un système de contrôle du PTT (temps de transit du pouls) permettant de mesurer l'éveil et les réponses à la tension ou à la douleur pendant la sédation ou l'anesthésie.

WO-A-2004 016 172 décrit une procédure de détection d'un état de stress par mesure en ambulatoire des battements du coeur puis définition dans une première étape de segments du signal de battements à l'aide d'une règle de segmentation choisie.

Objectifs de l'invention

La présente: invention a pour principal objectif de proposer une nouvelle solution technique de traitement automatique d'une série RR, qui pallie les inconvénients précités inhérents aux procédés de l'art antérieur basés sur une analyse spectral et qui permet le calcul d'une information quantitative (paramètre) caractérisant avec une très bonne sensibilité l'activité du SNA.

Résumé de l'invention

Cet objectif est atteint par le système de traitement de la revendication 1, et par le programme de traitement de la revendication 12.

Dans le présent texte, et notamment dans les revendications, on désigne par les termes « signals cardiaque », tout signal physique caractéristique du rythme (ou de la fréquence) cardiaque instantanée de l'être vivant Pour la mise en oeuvre de l'invention, différentes techniques invasives ou non invasives peuvent être utilisées pour acquérir ce signal cardiaque. Une technique invasive connue constate par exemple à utiliser un capteur de pression sanglante relié à un cathéter introduit dans une artère. Parmi les méthodes non invasives connues (et pour lesquelles on optera de préférence), on peut citer par exemple l'utilisation d'un capteur de pouls infrarouge, l'utilisation d'un capteur à ultrasons permettant la détection des cycles cardiaques, du type du capteur mis en oeuvre dans un cardiotocographe, ou encore l'acquisition d'un signal électrocardiographique (ECG). L'acquisition d'un signal électrocardiographique (ECG) est en pratique la méthode la plus couramment utilisée, car outre son caractère non invasif, elle permet d'obtenir un signal cardiaque plus précis que celui obtenu par exemple au moyen d'un capteur de pouls infrarouge.

Dans le présent texte, et notamment dans les revendications, on désigne d'une, manière générale par les termes « série RR », une série de plusieurs échantillons successifs $(RR_l)$, obtenus après échantillonnage d'un signal cardiaque analogique caractéristique du rythme cardiaque de l'être vivant, chaque échantillon (RRI) caractérisant d'une manière générale un intervalle de temps ($\delta$ti) entre deux battements cardiaques successifs ou l'inverse (1/$\delta$ti) de cet intervalle de temps.

Dans l'exemple préféré de réalisation décrit ci-après en référence aux figures annexée, cette série RR est plus particulièrement construite à partir des ondes R d'un signal ECG. Ceci n'est toutefois pas limitatif de l'invention. Dans le cas d'un signal cardiaque type ECG, on peut construire la série dite « RR » en utilisant les autres ondes de dépolarisation (P, Q, S ou T) du signal ECG pour construire la série RR, la précision étant toutefois moins bonne qu'en utilisant les ondes R du signal ECG. Egalement, lorsque le signal cardiaque n'est pas un signal ECG, les échantillons de la série RR ne sont pas calculés en déterminant l'intervalle de temps séparant deux ondes R successives du signal ECG, mais sont d'une manière plus générale déterminés en détectant dans le signal cardiaque l'intervalle de temps entre deux battements cardiaques successifs.

Le paramètre final calculé au moyen du programme ou du système de l'invention permet d'une manière générale de caractériser tout stimulus ayant un effet sur l'activité du SNA, et se traduisant par une variation du rythme (ou fréquence) cardiaque.

Une première application importante de l'invention se situe dans le domaine médical ou chirurgical, pour évaluer le niveau de douleur chez un être vivant

Dans le domaine médical ou chirurgical, il est en effet important de pouvoir connaître le niveau de douleur ressentie par un patient, afin notamment de pouvoir prendre en compte et traiter de manière optimale cette douleur.

La méthode d'évaluation de la douleur la plus répandue à ce jour est une méthode subjective, basée sur l'utilisation d'une réglette permettant au patient d'indiquer au moyen d'un curseur ou équivalent le niveau de douleur qu'il ressent sur une échelle de douleur pré-établie. Cette méthode présente l'inconvénient d'une part d'être purement subjective, et n'est donc pas réellement fiable, et d'autre part ne peut être envisagée qu'avec des patients conscients.

Au surplus, dans le cas d'un patient conscient sous anesthésie locale, tel que par exemple un patient sous péridurale, la méthode d'évaluation subjective précitée ne peut être utilisée pour contrôler automatiquement dans le temps l'administration des produits anesthésiques.

Dans le domaine de l'anesthésie générale, il est également intéressant de pouvoir connaître le niveau de douleur ressentie par le patient inconscient. La connaissance de ce niveau de douleur permet d'adapter au mieux l'administration des produits analgésiques.

Pour les raisons ci-dessus, il y a un intérêt important à proposer une méthode objective d'évaluation de la douleur ressentie par un être vivant, ladite méthode devant se traduire par un calcul automatique d'un paramètre mesurant le niveau de douleur.

Selon le cas, et grâce à l'invention, l'évaluation de la douleur peut être effectuée aussi bien sur un être vivant conscient et non ventilé mécaniquement (c'est-à-dire un être vivant dont la fréquence respiratoire est quelconque et variable, et n'est pas imposée par un dispositif de ventilation contrôlée par contraste notamment avec un patient sous anesthésie générale) ou sur un être vivant inconscient, et en particulier un être vivant sous anesthésie générale. Dans ce dernier cas (être vivant inconscient sous anesthésie générale), l'évaluation du niveau de douleur permet de connaître indirectement le niveau d'analgésie pendant l'anesthésie générale.

Une deuxième application importante de l'invention se situe dans le domaine paramédical, pour évaluer le niveau de stress chez un être vivant.

Description des figures

D'autre caractéristiques et avantages de l'invention apparaîtront plus clairement à la lecture de la description détaillée qui est donnée à titre d'exemple non limitatif et non exhaustif, et en référence aux dessins annexés sur lesquels :

- la figure 1 représente de manière schématique les principaux éléments d'un exemple d'un système d'analyse de l'invention,
- la figure 2 représente l'ensemble d'ondes (PQRST) caractéristique d'un battement cardiaque dans un signal ECG,
- la figure 3 représente un exemple de signal numérique ECG, obtenu après échantillonnage d'un signal ECG analogique,
- la figure 4 représente un exemple de série RR (encore désigné signal RR) construite à partir du signal de la figure 3, et
- la figure 5 représente un exemple de série RR après filtrage et normalisation.

Description détaillée

Système d'anayse de la variabilité du rythme cardiaque

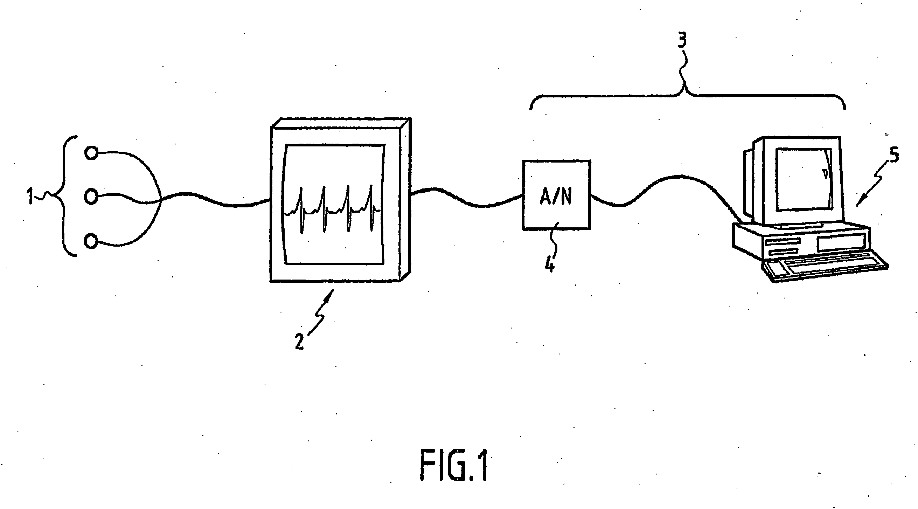

On a représenté sur la figure 1 un exemple de système d'analyse de la variabilité du rythme cardiaque qui permet l'acquisition et le traitement du signal cardiaque d'un être vivant (désigné par la suite « patient ») conformément à l'invention.

Ce système comporte :

- des moyens usuels d'acquisition d'un signal ECG, comprenant plusieurs électrodes de mesure 1 reliées en entrée à un moniteur électrocardiographique (ECG) 2,

- des moyens 3 de traitement du signal ECG délivré en sortie par le moniteur ECG 2.

Les moyens de traitement 3 du signal ECG comprennent un convertisseur analogique/numérique 4, et une unité de traitement programmée 5. L'entrée du convertisseur 4 est reliée à la sortie du moniteur ECG2, et la sortie du convertisseur 4 est reliée à un port d'entrée de l'unité de traitement 5. Dans un exemple particulier de réalisation, non limitatif de l'invention, l'unité de traitement 5 est constituée par un micro-ordinateur, le convertisseur 4 étant relié à un port de communication de ce micro-ordinateur (par exemple port série RS232 ou port USB).

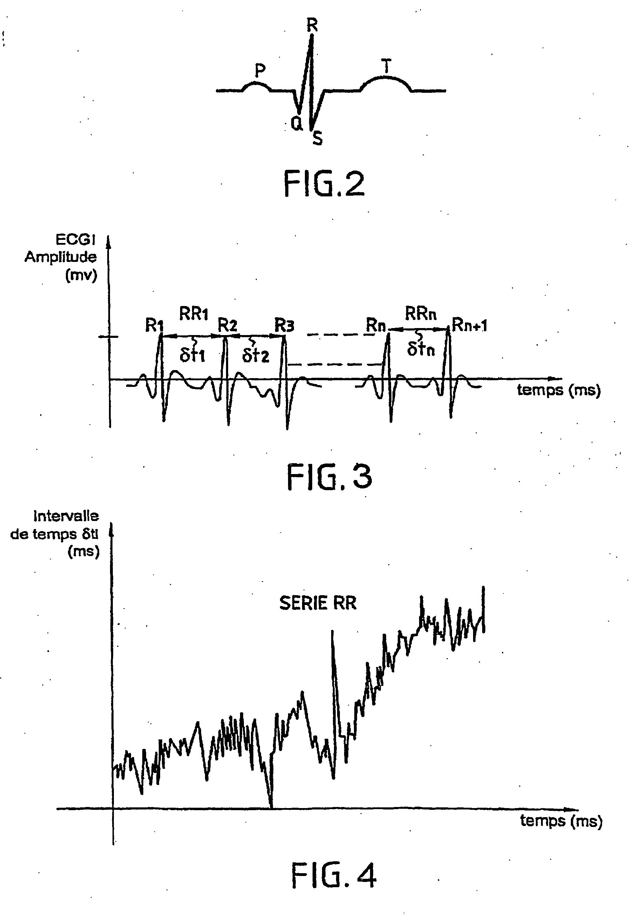

En fonctionnement, les électrodes 1 sont appliquées sur le corps d'un patient, et le moniteur ECG délivre en sortie de manière usuelle un signal électrique analogique, dit signal ECG, qui pour chaque battement cardiaque, a par exemple la forme du signal représenté a la figure 2.

En référence à la figure 2, pour chaque battement cardiaque, ce signal électrocardiographique (ECG) est constitué d'un ensemble d'ondes électriques :

- l'onde P, qui correspond à la dépolarisation des oreillettes, et qui présente une faible amplitude et une forme de dôme ;
- l'espace PQ qui traduit le temps de conduction auriculo-ventriculaire ;
- l'onde R considérée en pratique comme marqueur de la systole ventriculaire, ou du battement cardiaque, le complexe QRS reflétant la contraction ventriculaire, et
- l'onde T qui reflète la repolarisation ventriculaire.

Ce signal ECG analogique est numérisé par le convertisseur 4, avec une fréquence d'échantillonnage (fc) prédéterminée, valant par exemple 256 Hz.

Le signal échantillonné délivré en sortie du convertisseur 4, (signal représenté sur la figure 3) est traité par l'unité de traitement 5, au moyen d'un logiciel de traitement spécifique (logiciel d'évaluation de la douleur) qui est décrit en détail ultérieurement. Ce logiciel d'évaluation de la douleur est stocké en mémoire de l'unité de traitement 5, et permet, lorsqu'il est exécuté, de calculer automatiquement, à partir du signal numérique délivré par le convertisseur analogique/numérique 4, deux paramètres distincts (AUCmax et AUCmoy) mesurant le niveau de douleur du patient.

Une variante préférée de ce logiciel d'évaluation de la douleur va à présent être détaillée.

Exemple d'algorithme du logiciel d'évaluation de la douleur

Dans une variante préférée de réalisation de l'invention, les principales étapes successives de l'algorithme du logiciel d'évaluation de la douleur sont les suivantes :

1. Acquisition des échantillons RRi et ré-échantillonnage à une fréquence f prédéfinie
2. Sélection des échantillons RRi compris dans une fenêtre temporelle principale de n secondes (n>1/f)
3. Filtrage
4. Normalisation du signal
5. Détection des minimums (Pi) dans la fenêtre principale
6. Découpage de la fenêtre temporelle principale en m [m ≥2] sous-fenêtres ($F_j$) et calcul pour chaque sous-fenêtre ($F_j$) de l'aire ($A_j$) délimitée par l'enveloppe inférieure ($C_j$) reliant les minimums (Pi) détectés à l'étape précédente
7. Calcul des paramètres : AUCmoy et AUCmax
8. Décalage, d'un pas temporel valant p secondes (p≤n), de la fenêtre temporelle principale de n secondes, et réitération du calcul à partir de l'étape 2.

En pratique, le système peut être programmé pour être utilisé en temps réel ou en temps différé.

Lorsque le système est utilisé en temps différé, on réalise dans un premier temps l'étape 1 en temps réel en sorte d'acquérir tous les échantillons RRi sur toute la période d'analyse souhaitée ; l'intégralité de ces échantillons RRi successifs est stockée dans un fichier d'acquisition en mémoire de l'unité de traitement. Dans un second temps, les étapes 2 à 8 sont effectuées en boucle, en différé, sur les valeurs d'intervalle RRi stockées dans le fichier d'acquisition.

Lorsque le système fonctionne en temps réel, l'étape d'acquisition 1 d'une part, et les autres étapes de traitement 2 à 8 d'autre part sont exécutées par deux modules logiciels distincts fonctionnant en parallèle, le premier module d'acquisition (étape 1) alimentant le second module de traitement et de calcul (étapes 2 à 8) par l'intermédiaire d'un fichier tampon ou équivalent.

Les étapes 1 à 8 vont a présent être détaillées.

Etape 1 : Acquisition des échantillons RRi et ré-échantillonnage à une fréquence f prédéfinie

L'acquisition des échantillons RRi est réalisée par un premier sous-module logiciel qui est alimenté en entrée avec les données numériques successives constitutives du signal ECG numérisé (signal de la figure 3) délivré par le convertisseur analogique numérique 4. Chaque donnée (ou point) du signal ECG est définie par l'amplitude instantanée $ECG_i$ du signal ECG, et par l'instant $t_i$ d'échantillonnage ($t_i = n_i$ /fc, avec $n_i$ numéro d'échantillon et fc représentant la fréquence d'échantillonnage du convertisseur 4).

Le premier sous-module d'acquisition des échantillons $RR_i$ est conçu pour détecter automatiquement chaque pic Ri successif dans le signal numérique délivré par le convertisseur 4, et pour construire automatiquement une série RR (figure 4) constituée d'une succession de d'échantillons $RR_i$. Chaque échantillon $RR_i$ est défini par le couple de coordonnées : ti [un instant (ou numéro) d'échantillonnage] ; intervalle de temps δti (exprimé par exemple en multiple de la période d'échantillonnage fc) séparant un pic $R_i$ du pic suivant $R_{i+1}$(dans une autre variante il pourrait s'agir du pic précédent $R_{i-1}$).

De manière usuelle en soi, l'onde R étant le plus souvent la partie la plus fine et la plus ample du QRS, elle est de préférence utilisée pour détecter le battement cardiaque avec une très bonne précision, l'intervalle de temps δti correspondant en pratique au temps séparant deux battements cardiaques successifs. Néanmoins, dans une autre variante, on pourrait envisager d'utiliser d'autres ondes (par exemple onde Q ou onde S) d'un battement cardiaque du signal ECG pour détecter et construire la série RR.

La série RR (figure 4) délivrée par le premier sous-module précité est ré-échantillonnée automatiquement par un second sous-module logiciel à une fréquence prédéfinie f. Cette fréquence f de ré-échantillonnage doit être supérieure au double de la fréquence cardiaque maximale physiologique du patient. Afin de prendre en compte toutes les situations physiologiques possibles, la fréquence f de ré-échantillonnage est de préférence fixée à une valeur supérieure à 6Hz.

L'objectif de ce ré-échantillonnage est d'obtenir en sortie une série RR dont les échantillons $RR_i$ sont équidistants d'un point de vue temporel, c'est-à-dire en d'autres termes une série RR dont les instants d'échantillonnages sont réguliers. Ce ré-échantillonnage est réalisé de manière connue en soi par interpolation, et par exemple par interpolation linéaire.

Etape 2: Sélection des échantillons RRi compris dans une fenêtre temporelle principale de n secondes (n>1/f)

Cette étape revient à isoler un nombre N d'échantillons RRi successifs (N=n.f). A titre indicatif, on choisit par exemple une fenêtre principale de 64 secondes (n=64), ce qui correspond à 512 échantillons RRi successifs (N=512), pour une fréquence f de ré-échantillonnage de 8Hz.

Les étapes suivantes 3 à 7 sont appliquées aux échantillons compris dans cette fenêtre principale.

Etape 3 : filtrage / [f1 ; f2]

Cette étape consiste à appliquer un filtre passe-bande sur les échantillons de la série RR compris dans la fenêtre principale en sorte de conserver uniquement les fréquences comprises dans une bande de fréquence prédéfinie [f1 ; f2].

Plus particulièrement, la bande de fréquence [f1 ; f2] est égale ou est comprise dans la bande de fréquences [0,05Hz ; 5Hz]. De préférence, la bande de fréquence [f1 ; f2] est égale à [0,1 Hz ; 1 Hz].

Pour réaliser cette étape de filtrage passe-bande, on utilisera par exemple un filtre numérique passe-haut ayant une fréquence de coupure à la fréquence f1, en série avec un filtre numérique passe-bas ayant une fréquence de coupure à la fréquence f2. Il est également possible d'utiliser un filtre sélectif récursif à réponse impulsionnelle infinie (filtre RII) centré sur la bande de fréquence [f1 ;f2].

Le filtre passe haut (fréquence de coupure f1) a pour objectif de filtrer les basses fréquences inférieures à 0,1 Hz, et incidemment de supprimer de nombreux artefacts dans le signal. En pratique la fréquence de coupure f1 est donc supérieure ou égale à 0,1 Hz, et de préférence comprise entre 0,1 Hz et 0,15Hz. Il permet également avantageusement de supprimer la valeur moyenne du signal. Il est envisageable de ne pas réaliser de filtre passe-haut. Dans ce cas, il est préférable, avant de calculer les paramètres intermédiaires ($A_j$) de recentrer le signal sur sa moyenne.

Le filtre passe-bas (fréquence de coupure f2) a pour objectif de filtrer les hautes fréquences, typiquement supérieures à 1 Hz, car en pratique elles ne contiennent pas d'information intéressante. La mise en oeuvre de ce filtrage passe-bas, bien que préférentielle, est toutefois facultative et non indispensable à la réalisation de l'invention.

Etape 4 : Normalisation du signal

Cette étape est réalisée au moyen d'un sous-module logiciel qui dans un premier temps calcule la norme S du signal issu de l'étape 3 conformément à la formule usuelle ci-après :

$$S = \sqrt[p]{\sum_{i=1}^{N}(s_i)^p}$$

Où p est un entier supérieur à 1, et $s_i$ sont les valeurs discrètes du signal.

Dans une autre variante, la norme peut également être calculée au moyen de la formule ci-après :

$$S = \frac{\sqrt[p]{\sum_{i=1}^{N}(s_i)^p}}{N}$$

De préférence, pour la mise en oeuvre de l'invention, on choisira p égal à 2.

Dans un deuxième temps, le sous-module logiciel effectue une normalisation du signal en divisant chaque valeur $s_i$ du signal par la norme S précédemment calculée.

Cette étape 4 permet d'obtenir une meilleure sensibilité sur le résultat final (sensibilité des paramètres AUCmoy et AUCmax mesurant le niveau de douleur).

Il est essentiel que cette normalisation soit effectuée sur toute la largeur de la fenêtre principale, c'est-à-dire en prenant en compte dans la normalisation les (N) échantillons RRi de la fenêtre principale. Par contraste, une normalisation effectuée sur chaque sous-fenêtre ($A_j$) serait totalement inefficace.

Etape 5 : détection des minimums dans la fenêtre principale

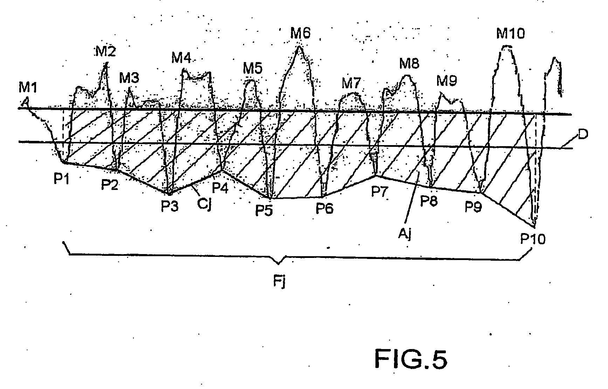

On a représenté sur la figure 5, un exemple de signal RR (après filtrage et normalisation) issu de l'étape 4 précitée. Sur cette figure, l'axe des abscisses correspond à la valeur nulle du signal filtré et normalisé.

L'étape 5 est réalisée au moyen d'un sous-module logiciel qui détecte les minimums (points $P_i$ sur la figure 5) de ce signal, par exemple au moyen d'un algorithme détectant une inversion de la pente (ou de manière équivalente un changement du signe de la dérivée du signal). Etape 6 : Découpage de la fenêtre temporelle principale en m [m ≥2] sous-fenêtres ($F_j$) et calcul pour chaque sous-fenêtre ($F_j$) de l'aire ($A_j$) délimitée par l'enveloppe inférieure ($C_j$), reliant les minimums détectés à l'étape précédente.

Le sous-module logiciel correspondant à l'étape 6 calcule automatiquement, pour chaque sous-fenêtre ($F_j$), une enveloppe inférieure (courbe $C_j$ sur la figure 5), qui relie les minimums (points $P_j$) détectés à l'étape 5 et compris dans la sous-fenêtre ($F_j$). Cette enveloppe inférieure ($C_j$) est par exemple déterminée par interpolation linéaire, c'est-à-dire en calculant les segments de droite reliant les points minimums ($P_i$).

Ensuite le sous-module logiciel correspondant à l'étape 6 calcule, pour chaque sous-fenêtre ($F_j$), l'aire ($A_j$) délimitée par l'enveloppe inférieure ($C_j$) et l'axe des abscisses (valeur nulle du signal) L'aire ($A_j$) correspond à la surface hachurée sur la figure 5.

Lorsque l'enveloppe inférieure ($C_j$) est approximée par des segments de droite, ce calcul d'aire ($A_j$) revient à calculer la somme des aires des trapèzes délimités par deux point minimum ($P_i$) successifs et l'axe des abscisses.

Dans une autre variante de réalisation, l'aire ($A_j$) pourrait être délimitée par l'enveloppe inférieure ($C_j$) et une droite horizontale différente de l'axe des abscisses, et de préférence (mais non nécessairement) positionnée sous l'axe des abscisses, tel que par exemple la droite D en pointillés sur la figure 5. Egalement, dans une autre variante, cette droite D pour le calcul de l'aire ($A_j$) pourrait être située au dessus de l'axe des abscisses, et notamment passer par le point $M_i$ de plus grande ordonnée (point M10 dans l'exemple particulier de la figure 5).

A titre d'exemple non limitatif, le procédé a été testé avec une durée de fenêtre principale valant 64s, et pour la mise en oeuvre de l'étape 6, cette fenêtre principale était découpée en quatre (m=4) sous-fenêtres $F_1$ à $F_4$ de 16s.

Etape 7 : Calcul des paramètres : AUCmoy et AUCmax

Les deux paramètres AUCmoy et AUCmax permettent de mesurer le niveau de douleur.

AUCmoy est égal (ou plus généralement proportionnel) à la moyenne des aires Aj des (m) sous-fenêtrés $F_j$.

AUCmax est égal (ou plus généralement proportionnel) à la valeur de la plus grande des aires Aj calculées pour les (m) sous-fenêtres $F_j$. Etape 8 : Décalage, d'un pas temporel valant p secondes (p ≤ n), de la fenêtre temporelle de n secondes, et réitération du calcul à partir de l'étape 2.

Le pas (p) pour le glissement de la fenêtre principal de calcul et réitération des étapes 2 à 8 influe sur la sensibilité des paramètres AUCmoy et AUCmax, et dépend donc de la sensibilité souhaitée. A titre indicatif, et en pratique, des résultats très satisfaisants ont été obtenus en choisissant un pas (p) valant 2s.

Dans une autre variante de réalisation, l'étape de filtrage précitée (Etape 3) pourrait être effectuée avant l'étape 2 de sélection, par exemple en continu au fur et à mesure de l'acquisition des échantillons RRi. Egalement, dans une autre variante de réalisation, cette étape de filtrage pourrait être réalisée après l'étape de normalisation (étape 4 précitée).

Validation expérimentale

Des essais ont montré que le paramètre AUCmoy et le paramètre AUCmax étaient chacun corrélés à l'intensité du stimulus douloureux ressenti par un être vivant, ces paramètres permettant ainsi de mesurer l'intensité de la douleur chez un être vivant.

Afin de valider cette corrélation entre les paramètres précités AUCmoy et AUCmax et le niveau de douleur ressenti, ces paramètres AUCmoy et AUCmax ont été testés sur une population de parturientes majeures, sans antécédent cardio-vasculaire, diabétique ou neurologique, classées ASA I ou II, devant accoucher à terme et par voie basse sous analgésie péridurale (APD).

Les valeurs calculées AUCmoy et AUCmax ont été comparées avec les indications de niveau de douleur fournies par les parturientes au moyen d'une échelle visuelle analogique de la douleur (EVA), avant mise en place de la péridurale et après mise en place de la péridurale.

Cette comparaison a permis de démontrer que les paramètres AUCmoy et AUCmax étaient corrélés avec les indications de niveau de douleur fournies par les parturientes au moyen de l'EVA.

Il ressort plus particulièrement que le paramètre AUCmax est le paramètre le plus fiable, et est donc préférentiel.

Les deux paramètres AUCmax et AUCmoy ont été testés sur des patients conscients, et on a pu constater que de manière avantageuse selon l'invention, ces paramètres sont indépendants de la fréquence respiratoire propre de l'être vivant.

Les deux paramètres AUCmax et AUCmoy ont également été testés avec succès pour l'évaluation de la douleur sur des patients non conscients, sous anesthésie générale dont la fréquence respiratoire était imposée par un mécanisme de ventilation contrôlée. La connaissance en anesthésie générale de ces paramètres permet indirectement de mesurer la composante analgésique de l'anesthésie générale.

L'invention n'est toutefois pas limitée aux deux paramètres particuliers précités AUCmax et AUCmoy, qui ont été donnés uniquement à titre d'exemples préférés.

Plus généralement, il a été constaté qu'un élément essentiel de l'invention influant sur le résultat visé (évaluation de la douleur) était le calcul d'un paramètre final à partir de paramètres intermédiaires calculés sur les échantillons RRi compris dans des sous-fenêtre temporelles, par contraste avec une solution (non couverte par l'invention) dans laquelle le paramètre final serait calculé directement sur l'ensemble des échantillons RRi de la fenêtre principale. Dans sa généralité, l'invention pourra donc être mis en oeuvre avec toute méthode de traitement des échantillons RRi permettant de calculer un paramètre intermédiaire Aj dans des sous-fenêtres temporelles, le paramètre final corrélé avec l'intensité du stimulus douloureux, étant fonction de ces paramètres intermédiaires.

Egalement, il a été mis en évidence que de manière inattendue le stimulus douloureux influait très sensiblement sur les pics respiratoires (points minimum Pi) ; plus l'intensité de la douleur est importante, et plus l'amplitude des pics dans le signal correspondant aux minimums (Pi) est faible, et peut dans certains cas être quasiment nulle. En revanche, les maximums (Mi) de la courbe ne sont pas sensiblement affectés par le stimulus douloureux. En conséquence, toute méthode de traitement des échantillons RRi dans chaque sous-fenêtre (Fj) permettant de prendre en compte les variations d'amplitude de ces pics (Pi) sera appropriée pour calculer les paramètre intermédiaires dans chaque sous-fenêtre, et l'invention n'est donc pas limitée au calcul d'aire précédemment décrit et donné à titre uniquement d'exemple. A titre d'exemple non exhaustifs, le calcul des aires (Aj) pourrait être remplacé par le calcul d'un paramètre intermédiaire fonction des amplitudes des pics correspondant aux points minimum (Pi). Egalement , dans une autre variante de réalisation, il est possible de calculer chaque paramètre intermédiaire ($A_j$) en appliquant sur les échantillons RRi de la sous-fenêtre ($F_j$) une transformée en ondelettes, basée sur une ondelette de forme appropriée (par exemple ondelette de Daubechies).

Dans les exemples de réalisation qui ont été décrits en référence aux figures annexées, les algorithmes de calcul sont appliqués sur une série RR dont les échantillons (RRi) caractérisent les intervalles de temps ($\delta$ti) qui séparent deux battements cardiaques successifs. Dans une autre variante de réalisation, l'invention peut être mise en oeuvre à partir d'une série RR dont les échantillons (RRi) caractérisent l'inverse (1/δti) des intervalles de temps (δti) entre deux battements cardiaques successifs. Dans ce cas, en appliquant les étapes 1 à 4 précitées, on obtient une courbe inverse de celle de la figure 5, les point Pi devenant des maximums et les point Mi devenant des minimums, et le calcul des aires (Aj) précédemment décrit (étape 6) est réalisé à partir d'une enveloppe supérieure (Cj) reliant les points maximums (Pi).

L'algorithme de calcul qui vient d'être décrit en détail, ainsi que les paramètres qui en découlent, notamment les paramètres AUCmoy et AUCmax, ne sont pas limités en termes d'application à l'évaluation de la douleur chez un être vivant, mais peuvent d'une manière plus générale être utilisés pour mesurer la variabilité du rythme cardiaque chez un être vivant (conscient ou inconscient), et de ce fait pour étudier les effets sur le SNA (Système Nerveux Autonome) de tout stimulus susceptible de modifier l'activité du SNA.

Ainsi, l'enseignement précité appliqué à l'évaluation de la douleur, peut être également utilisé et transposé tel quel dans d'autre domaines d'applications, et en particulier dans le domaine paramédical, pour évaluer le niveau de stress ressenti par un être vivant (conscient ou inconscient).

**Revendications**

1. Système d'analyse de la variabilité du rythme cardiaque, ledit système comportant des moyens (1, 2) d'acquisition d'un signal cardiaque analogique, des moyens (4) d'échantillonnage de ce signal cardiaque, et des moyens (5) de traitement du signal échantillonné conçus pour construire une série RR constituée d'une pluralité d'échantillons (RR$_i$) représentant les intervalles de temps (δ$_{ti}$) qui séparent deux battements cardiaques successifs ou l'inverse (1/δ$_{ti}$) de ces intervalles de temps,

   lesdits moyens de traitement (5) sont en outre conçus pour :

   > sélectionner (N) échantillons (RR$_i$) dans une fenêtre temporelle principale de durée (n) prédéfinie,
   > découper cette fenêtre principale en (m) sous-fenêtres (F$_j$), chaque sous-fenêtre (F$_j$) comportant plusieurs échantillons (RR$_i$),
   > calculer pour chaque sous-fenêtre (F$_j$) un paramètre intermédiaire (A$_j$) à partir des échantillons (RR$_i$) compris dans la sous-fenêtre (F$_j$), et calculer un paramètre final qui est fonction des paramètres intermédiaires (A$_j$).

2. Système selon la revendication 1, où lesdits moyens de traitement (5) sont conçus pour calculer de manière itérative le paramètre final en décalant la fenêtre temporelle principale d'un pas de durée (p) prédéfinie inférieure à la durée (n) de la fenêtre principale.

3. Système selon la revendication 1 ou 2, où lesdits moyens de traitement (5) sont conçus pour calculer chaque paramètre intermédiaire (A$_j$) en fonction au moins des minimums (P$_i$) de la série RR dans la sous-fenêtre (F$_j$) lorsque les échantillons (RR$_i$) de la série (RR) représentent les intervalles de temps (δ$_{ti}$) qui séparent deux battements cardiaques successifs, ou en fonction au moins des maximums (P$_i$) de la série RR dans la sous-fenêtre (F$_j$) lorsque les échantillons (RR$_i$) de la série (RR) représentent l'inverse (1/δ$_{ti}$) des intervalles de temps (δ$_{ti}$) qui séparent deux battements cardiaques successifs.

4. Système selon l'une des revendications 1 à 3, où la valeur calculée pour le paramètre final (AUC$_{max}$) est proportionnelle à la valeur maximale des paramètres intermédiaires (A$_j$).

5. Système selon l'une des revendications 1 à 3, où la valeur calculée pour le paramètre final (AUC$_{moy}$) est proportionnelle à la moyenne des valeurs des paramètres intermédiaires (A$_j$).

6. Système selon l'une des revendications 1 à 5, où calculer les paramètres intermédiaires (A$_j$) comporte déterminer une enveloppe (C$_j$), reliant les points minimums (P$_i$) détectés lorsque les échantillons (RR$_i$) de la série (RR) représentent les intervalles de temps (δ$_{ti}$) qui séparent deux battements cardiaques successifs, ou reliant les points maximums (P$_i$) détectés lorsque les échantillons (RR$_i$) de la série (RR) représentent l'inverse (1/δ$_{ti}$) des intervalles de temps (δ$_{ti}$) qui séparent deux battements cardiaques successifs.

7. Système selon la revendication 6, où chaque paramètre intermédiaire (A$_j$) est fonction d'une aire délimitée par l'enveloppe (C$_j$).

8. Système selon l'une quelconque des revendications 1 à 7, où lesdits moyens de traitement (5) sont pour, préalablement au calcul du paramètre, filtrer la série RR au moyen d'un filtre passe-haut présentant une fréquence de coupure f1 supérieure ou égale à 0,1 Hz, et de préférence comprise entre 0,1 Hz et 0,15 Hz.

9. Système selon l'une quelconque des revendications 1 à 8, où lesdits moyens de traitement (5) sont conçus pour, préalablement au calcul des paramètres intermédiaires (A$_j$) dans chaque sous fenêtre (F$_j$), normaliser les échantillons (RR$_i$) de la série RR sur toute la largeur de la fenêtre principale.

**10.** Système selon la revendication 1 pour l'évaluation de la douleur, le paramètre final calculé caractérisant un niveau de douleur.

**11.** Système selon la revendication 1 pour l'évaluation du stress, le paramètre final calculé caractérisant un niveau de stress.

**12.** Programme apte à être exécuté automatiquement par les moyens de traitement du système selon la revendication 1 et permettant le traitement d'une série RR constituée d'une pluralité d'échantillons $(RR_i)$ représentant les intervalles de temps $(\delta_{ti})$ qui séparent deux battements cardiaques successifs ou l'inverse $(1/\delta_{ti})$ de ces intervalles de temps, lorsque il est exécuté par lesdits moyens de traitement, il amène lesdits moyens de traitement à:

sélectionner (N) échantillons $(RR_i)$ dans une fenêtre temporelle principale de durée (n) prédéfinie,
découper cette fenêtre principale en (m) sous-fenêtres $(F_j)$, chaque sous-fenêtre $(F_j)$ comportant plusieurs échantillons $(RR_i)$,
calculer pour chaque sous-fenêtre $(F_j)$ un paramètre intermédiaire $(A_j)$ à partir des échantillons $(RR_i)$ compris dans la sous-fenêtre $(F_j)$, et
calculer un paramètre final qui est fonction des paramètres intermédiaires $(A_j)$.

**13.** Programme selon la revendication 12, où il amène lesdits moyens de traitement à calculer de manière itérative le paramètre final en décalant la fenêtre temporelle principale d'un pas de durée (p) prédéfinie inférieure à la durée (n) de la fenêtre principale.

**14.** Programme selon la revendication 12 ou 13, où il amène lesdits moyens de traitement à calculer chaque paramètre intermédiaire $(A_j)$ en fonction au moins des minimums $(P_i)$ de la série RR dans la sous-fenêtre $(F_j)$ lorsque les échantillons $(RR_i)$ de la série (RR) représentent les intervalles de temps $(\delta_{ti})$ qui séparent deux battements cardiaques successifs, ou en fonction au moins des maximums $(P_i)$ de la série RR dans la sous-fenêtre $(F_j)$ lorsque les échantillons $(RR_i)$ de la série (RR) représentent l'inverse $(1/\delta_{ti})$ des intervalles de temps $(\delta_{ti})$ qui séparent deux battements cardiaques successifs.

**15.** Programme selon l'une des revendications 12 à 14 où la valeur calculée pour le paramètre final $(AUC_{max})$ est proportionnelle à la valeur maximale des paramètres intermédiaires $(A_j)$.

**16.** Programme selon l'une des revendications 12 à 14, où la valeur calculée pour le paramètre final $(AUC_{moy})$ est proportionnelle à la moyenne des valeurs des paramètres intermédiaires $(A_j)$.

**17.** Programme selon l'une des revendications 12 à 16, où calculer les paramètres intermédiaires $(A_j)$ comporte déterminer une enveloppe $(C_j)$, reliant les points minimums $(P_i)$ détectés lorsque les échantillons $(RR_i)$ de la série (RR) représentent les intervalles de temps $(\delta_{ti})$ qui séparent deux battements cardiaques successifs, ou reliant les points maximums $(P_i)$ détectés lorsque les échantillons $(RR_i)$ de la série (RR) représentent l'inverse $(1/\delta_{ti})$ des intervalles de temps $(\delta_{ti})$ qui séparent deux battements cardiaques successifs.

**18.** Programme selon la revendication 17, où chaque paramètre intermédiaire $(A_j)$ est fonction d'une aire délimitée par l'enveloppe $(C_j)$.

**19.** Programme selon l'une quelconque des revendications 12 à 18, où il amène lesdits moyens de traitement à, préalablement au calcul du paramètre, filtrer la série RR au moyen d'un filtre passe-haut présentant une fréquence de coupure f1 supérieure ou égale à 0,1 Hz, et de préférence comprise entre 0,1 Hz et 0,15Hz.

**20.** Programme selon l'une quelconque des revendications 12 à 19, où il amène lesdits moyens de traitement à, préalablement au calcul des paramètres intermédiaires $(A_j)$ dans chaque sous fenêtre $(F_j)$, normaliser les échantillons $(RR_i)$ de la série RR sur toute la largeur de la fenêtre principale.

**21.** Programme selon l'une quelconque des revendications 12 à 20, pour l'évaluation de la douleur ressentie par un être vivant, le paramètre final calculé par le programme caractérisant le niveau de douleur.

**22.** Programme selon l'une quelconque des revendications 12 à 20, pour l'évaluation du stress ressenti par un être vivant, le paramètre final calculé par le programme caractérisant le niveau de stress.

**Patentansprüche**

**1. System zum Analysieren** der Herzfrequenzvariabilität, wobei das System Erfassungsmittel (1, 2) zum Erfassen eines analogen Herzsignals, Abtastmittel (4) zum Abtasten dieses Herzsignals und Verarbeitungsmittel (5) zum Verarbeiten des abgetasteten Signals aufweist, die dazu ausgelegt sind, eine RR-Serie zu erstellen, die aus einer Mehrzahl von Abtastwerten $(RR_i)$ besteht, die Zeitintervalle $(\delta_{ti})$ darstellen, welche zwei aufeinanderfolgende Herzschläge voneinander trennen, oder den Kehrwert $(1/\delta_{ti})$ dieser Zeitintervalle darstellen, wobei die Verarbeitungsmittel (5) ferner dazu ausgelegt sind, aus einem Hauptzeitfenster mit vorbestimmter Zeit-

dauer (n) Abtastwerte ($RR_i$] auszuwählen (N), dieses Hauptfenster in (m) Unterfenster ($F_j$) zu zerlegen, wobei jedes Unterfenster ($F_j$) mehrere Abtastwerte ($RR_i$) enthält, für jedes Unterfenster ($F_j$) einen Zwischenparameter ($A_j$), ausgehend von den Abtastwerten ($RR_i$) zu berechnen, die im Unterfenster ($F_j$) enthalten sind, und einen Endparameter zu berechnen, der eine Funktion von den Zwischenparametern ($A_j$) ist.

2. System nach Anspruch 1, wobei die Verarbeitungsmittel (5) dazu ausgelegt sind, den Endparameter iterativ zu berechnen, indem das Hauptzeitfenster um einen Schritt vorbestimmter Zeitdauer (p) verschoben wird, die kürzer ist als die Zeitdauer (n) des Hauptfensters.

3. System nach Anspruch 1 oder 2, wobei die Verarbeitungsmittel (5) dazu ausgelegt sind, jeden Zwischenparameter ($A_j$) in Abhängigkeit zumindest von den Mindestwerten ($P_i$) der RR-Serie in dem Unterfenster ($F_j$) dann zu berechnen, wenn die Abtastwerte ($RR_i$) der RR-Serie Zeitintervalle ($\delta_{ti}$) darstellen, die zwei aufeinanderfolgende Herzschläge voneinander trennen, oder aber in Abhängigkeit zumindest von den Höchstwerten ($P_i$) der RR-Serie in dem Unterfenster ($F_j$) dann zu berechnen, wenn die Abtastwerte ($RR_i$) der RR-Serie den Kehrwert ($1/\delta_{ti}$) der Zeitintervalle ($\delta_{ti}$) darstellen, die zwei aufeinanderfolgende Herzschläge voneinander trennen.

4. System nach einem der Ansprüche 1 bis 3, wobei der für den Endparameter berechnete Wert ($AUC_{max}$) proportional ist zum Max- oder Höchstwert der Zwischenparameter ($A_j$).

5. System nach einem der Ansprüche 1 bis 3, wobei der für den Endparameter berechnete Wert ($AUC_{moy}$) proportional ist zum Durchschnittswert der Werte der Zwischenparameter ($A_j$).

6. System nach einem der Ansprüche 1 bis 5, wobei das Berechnen der Zwischenparameter ($A_j$) das Bestimmen einer Hüllkurve ($C_j$) umfasst, welche die erfassten Mindestpunkte ($P_i$) dann verbindet, wenn die Abtastwerte ($RR_i$) der RR-Serie die Zeitintervalle ($\delta_{ti}$) darstellen, die zwei aufeinanderfolgende Herzschläge voneinander trennen, oder die erfassten Höchstpunkte ($P_i$) dann verbindet, wenn die Abtastwerte ($RR_i$) der RR-Serie den Kehrwert ($1/\delta_{ti}$) der Zeitintervalle ($\delta_{ti}$) darstellen, die zwei aufeinanderfolgende Herzschläge voneinander trennen.

7. System nach Anspruch 6, wobei jeder Zwischenparameter ($A_j$) Funktion einer von der Hüllkurve ($C_j$) begrenzten Fläche ist.

8. System nach einem der Ansprüche 1 bis 7, wobei die Verarbeitungsmittel (5) dazu ausgelegt sind, vor dem Berechnen des Parameters die RR-Serie mittels eines Hochpassfilters zu filtern, das eine Grenzfrequenz f1 aufweist, die höher oder gleich 0,1Hz ist und vorzugsweise zwischen 0,1Hz und 0,15Hz liegt.

9. System nach einem der Ansprüche 1 bis 8, wobei die Verarbeitungsmittel (5) dazu ausgelegt sind, vor dem Berechnen der Zwischenparameter ($A_j$) in jedem Unterfenster ($F_j$) die Abtastwerte ($RR_i$) der RR-Serie über die gesamte Größe oder Breite des Hauptfensters zu normieren.

10. System nach Anspruch 1 zum Bewerten von Schmerz, wobei der errechnete Endparameter einen Schmerzpegel kennzeichnet.

11. System nach Anspruch 1 zum Bewerten von Stress, wobei der errechnete Endparameter ein Stressniveau oder Stresspegel kennzeichnet.

12. **Programm,** eingerichtet automatisch von den Verarbeitungsmitteln des Systems nach Anspruch 1 ausgeführt zu werden und das Verarbeiten einer RR-Serie ermöglicht, die aus einer Mehrzahl von Abtastwerten ($RR_i$) besteht, die Zeitintervalle ($\delta_{ti}$) darstellen, welche zwei aufeinanderfolgende Herzschläge voneinander trennen, oder den Kehrwert ($1/\delta_{ti}$) dieser Zeitintervalle darstellen, wobei es bei Ausführung durch die Verarbeitungsmittel, diese dazu veranlasst, aus einem Hauptzeitfenster vorbestimmter Zeitdauer (n) Abtastwerte ($RR_i$) auszuwählen (N), dieses Hauptfenster in (m) Unterfenster ($F_j$) zu zerlegen, wobei jedes Unterfenster ($F_j$) mehrere Abtastwerte ($RR_i$) aufweist, für jedes Unterfenster ($F_j$) einen Zwischenparameter ($A_j$), ausgehend von den Abtastwerten ($RR_i$) zu berechnen, die im Unterfenster ($F_j$) enthalten sind, und einen Endparameter zu berechnen, der eine Funktion von den Zwischenparametern ($A_j$) ist.

13. Programm nach Anspruch 12, wobei es die Verarbeitungsmittel dazu veranlasst, den Endparameter iterativ zu berechnen, indem das Hauptzeitfenster um einen Schritt vorbestimmter Zeitdauer (p) verschoben wird, die kürzer ist als die Zeitdauer (n) des Hauptfensters.

14. Programm nach Anspruch 12 oder 13, wobei es die Verarbeitungsmittel dazu veranlasst, jeden Zwischenparameter ($A_j$) in Abhängigkeit zumindest von den Mindestwerten ($P_i$) der RR-Serie in dem Unterfenster ($F_j$) dann zu berechnen, wenn die Abtastwerte (RRi) der RR-Serie Zeitintervalle ($\delta_{ti}$] darstellen, die zwei aufeinanderfolgende Herzschläge voneinander trennen, oder aber in Abhängigkeit zumindest von den Höchstwerten ($P_i$) der RR-Serie in dem Un-

terfenster ($F_j$) dann zu berechnen, wenn die Abtastwerte ($RR_i$) der RR-Serie den Kehrwert ($1/\delta_{ti}$) der Zeitintervalle ($\delta_{ti}$) darstellen, die zwei aufeinanderfolgende Herzschläge voneinander trennen.

15. Programm nach einem der Ansprüche 12 bis 14, wobei der für den Endparameter errechnete Wert ($AUC_{max}$) proportional ist zum Höchstwert der Zwischenparameter ($A_j$).

16. Programm nach einem der Ansprüche 12 bis 14, wobei der für den Endparameter errechnete Wert ($AUC_{moy}$] proportional ist zum Durchschnittswert der Werte der Zwischenparameter ($A_j$).

17. Programm nach einem der Ansprüche 12 bis 16, wobei das Berechnen der Zwischenparameter ($A_j$) das Bestimmen einer Hüllkurve ($C_j$) umfasst, welche die erfassten Mindestpunkte ($P_i$) dann verbindet, wenn die Abtastwerte ($RR_i$) der RR-Serie die Zeitintervalle ($\delta_{ti}$) darstellen, die zwei aufeinanderfolgende Herzschläge voneinander trennen, oder die erfassten Höchstpunkte ($P_i$) dann verbindet, wenn die Abtastwerte ($RR_i$) der RR-Serie den Kehrwert ($1/\delta_{ti}$) der Zeitintervalle ($\delta_{ti}$) darstellen, die zwei aufeinanderfolgende Herzschläge voneinander trennen.

18. Programm nach Anspruch 17, wobei jeder Zwischenparameter ($A_j$) eine Funktion einer von der Hüllkurve ($C_j$) begrenzten Fläche ist.

19. Programm nach einem der Ansprüche 12 bis 18, wobei es die Verarbeitungsmittel dazu veranlasst, vor dem Berechnen des Parameters die RR-Serie mittels eines Hochpassfilters zu filtern, das eine Grenzfrequenz f1 aufweist, die höher oder gleich 0,1Hz ist und vorzugsweise zwischen 0,1Hz und 0,15Hz liegt.

20. Programm nach einem der Ansprüche 12 bis 19, wobei es die Verarbeitungsmittel dazu veranlasst, vor dem Berechnen der Zwischenparameter ($A_j$) in jedem Unterfenster ($F_j$) die Abtastwerte ($RR_i$) der RR-Serie über die gesamte Größe oder Breite des Hauptfensters zu normieren.

21. Programm nach einem der Ansprüche 12 bis 20 zum Bewerten des von einem Lebewesen empfundenen Schmerzes, wobei der von dem Programm errechnete Endparameter den Schmerzpegel kennzeichnet.

22. Programm nach einem der Ansprüche 12 bis 20 zum Bewerten des von einem Lebewesen empfundenen Stress, wobei der von dem Programm errechnete Endparameter das Stressniveau oder den Stresspegel kennzeichnet.

**Claims**

1. System for analyzing the variability of the cardiac rhythm, said system comprising means (1,2) for acquiring an analogue cardiac signal, means (4) for sampling said cardiac signal, and processing means (5) for processing the sampled signal, said processing means (5) being designed to construct an RR series consisting of a plurality of samples ($RR_i$) representing the time intervals ($\delta t_i$) separating two successive heart beats or the inverse ($1/\delta t_i$) of said time intervals,

said processing means (5) being further designed for:

selecting (N) samples ($RR_i$) in a main time window having a predetermined length of time (n), cutting said main window into (m) subwindows ($F_j$), each subwindow ($F_j$) comprising several samples ($RR_i$)

calculating an intermediary parameter ($A_j$) for each subwindow ($F_j$) on the basis of the samples ($RR_i$) contained in the subwindow ($F_j$) and calculating a final parameter as a function of the intermediary parameters ($A_j$).

2. System according to claim 1, wherein said processing means (5) are designed to calculate the final parameter by iteration, by shifting the main time window by a predetermined time interval (p) less than the length of time (n) of the main window.

3. System according to claim 1 or 2, wherein said processing means (5) are designed to calculate each intermediary parameter ($A_j$) as function at least of the minima ($P_i$) of the RR series in the subwindow ($F_j$) when the samples ($RR_i$) of the series (RR) represent the time intervals ($\delta t_i$) separating two successive heart beats, or as a function at least of the maxima ($P_i$) of the RR series in the subwindow ($F_j$) when the samples ($RR_i$) of the series (RR) represent the inverse ($1/\delta t_i$) of the time intervals separating two successive heart beats.

4. System according to any one of claims 1 to 3, wherein the calculated value for the final parameter ($AUC_{max}$) is proportional to the maximum value of the intermediary parameters ($A_j$).

5. System according to any one of claims 1 to 3, wherein the calculated value for the final parameter ($AUC_{moy}$) is proportional to the average value of the intermediary parameters ($A_j$).

6. System according to any one of claims 1 to 5, wherein for the calculation of the intermediary parameters ($A_j$), an envelope ($C_j$) is defined, linking the minimum points ($P_i$) measured when the samples ($RR_i$) of the

series (RR) represent the time intervals ($\delta t_i$) separating two successive heart beats, or linking the maximum points ($P_i$) measured when the samples ($RR_i$) of the series (RR) represent the inverse ($1/\delta t_i$) of the time intervals separating two successive heart beats.

7. System according claim 6, wherein each intermediary parameter ($A_j$) is a function of an area delimited by the envelope ($C_j$).

8. System according to any one of claims 1 to 7, wherein said processing means (5) are designed to filter the RR series, prior to the calculation of the parameter, by means of a high-pass filter with a cut-off frequency f1 superior or equal to 0.1 Hz, and preferably lying between 0.1 Hz and 0.15 Hz.

9. System according to any one of claims 1 to 8, wherein said processing means (5) are designed to normalize the samples ($RR_i$) of the RR series are normalized over the entire width of the main window, prior to the calculation of the intermediary parameters ($A_j$) in each subwindow ($F_j$).

10. System according to claim 1 for the assessment of pain, the final calculated parameter characterising a level of pain.

11. System according to claim 1 for the assessment of stress, the final calculated parameter characterising a level of stress.

12. A program that can be automatically executed by the process means of claim 1, and that is capable to process a RR series consisting of a plurality of samples ($RR_i$) representing the time intervals ($\delta t_i$) separating two successive heart beats or the inverse ($1/\delta t_i$) of said time intervals, and once executed by said process means, said program enabling said process means to:

  select (N) samples ($RR_i$) in a main time window having a predetermined length of time (n),
  cut said main window into (m) subwindows ($F_j$), each subwindow ($F_j$) comprising several samples ($RR_i$)
  calculate an intermediary parameter ($A_j$) for each subwindow ($F_j$) on the basis of the samples ($RR_i$) contained in the subwindow ($F_j$) and calculate a final parameter as a function of the intermediary parameters ($A_j$).

13. A program according to claim 12, enabling said processing means to calculate the final parameter by iteration, by shifting the main time window by a predetermined time interval (p) less than the length of time (n) of the main window.

14. A program according to claim 12 or 13, enabling said processing means to calculate each intermediary parameter ($A_j$) as function at least of the minima ($P_i$) of the RR series in the subwindow ($F_j$) when the samples ($RR_i$) of the series (RR) represent the time intervals ($\delta t_i$) separating two successive heart beats, or as a function at least of the maxima ($P_i$) of the RR series in the subwindow ($F_j$) when the samples ($RR_i$) of the series (RR) represent the inverse ($1/\delta t_i$) of the time intervals separating two successive heart beats.

15. A program according to any one of claims 12 to 14, wherein the calculated value for the final parameter ($AUC_{max}$) is proportional to the maximum value of the intermediary parameters ($A_j$).

16. A program according to any one of claims 12 to 14, wherein the calculated value for the final parameter ($AUC_{moy}$) is proportional to the average value of the intermediary parameters ($A_j$).

17. A program according to any one of claims 12 to 16, wherein for the calculation of the intermediary parameters ($A_j$), an envelope ($C_j$) is defined, linking the minimum points ($P_i$) measured when the samples ($RR_i$) of the series (RR) represent the time intervals ($\delta t_i$) separating two successive heart beats, or linking the maximum points ($P_i$) measured when the samples ($RR_i$) of the series (RR) represent the inverse ($1/\delta t_i$) of the time intervals ($\delta t_i$) separating two successive heart beats.

18. A program according to claim 17, wherein each intermediary parameter ($A_j$) is a function of an area delimited by the envelope ($C_j$).

19. A program according to any one of claims 12 to 18, enabling said processing means (5) to filter the RR series, prior to the calculation of the parameter, by means of a high-pass filter with a cut-off frequency f1 superior or equal to 0.1 Hz, and preferably lying between 0.1 Hz and 0.15 Hz.

20. A program according to any one of claims 12 to 19, enabling said processing means to normalize the samples ($RR_i$) of the RR series are normalized over the entire width of the main window, prior to the calculation of the intermediary parameters ($A_j$) in each subwindow ($F_j$).

21. A program according to any one of claims 12 to 20, for the assessment of the pain felt by a living being, the final calculated parameter characterising a level of pain.

22. A program according to any one of claims 12 to 20, for the assessment of the stress felt by a living being, the final calculated parameter characterising a level of stress.

**FIG.1**

EP 1 804 655 B1

FIG.2

FIG.3

FIG.4

FIG.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03057034 A
- WO 03084396 A
- WO 2004016172 A

**Littérature non-brevet citée dans la description**

- **LACROIX D ; LOGIER R. ; KACET S. ; HAZARD J-R ; DAGANO J.** Effects of consécutive administration of central and peripheral anticholinergic agents on respiratory sinus arrhythmia in normal subjects. *J. of the Autonomic Nervous System,* 1992, vol. 39, 211-218